# EUROPEAN PATENT SPECIFICATION

(11) **EP 2 704 760 B1**
(45) Date of publication and mention of the grant of the patent: **20.09.2017**
(21) Application number: 12779892.4
(22) Date of filing: 02.05.2012
(51) Int. Cl.: A01N 1/02, C12M 1/00, A61M 1/02

(54) **PLATELET RESUSPENSION METHOD AND APPARATUS**
BLUTPLÄTTCHENAUFLOCKERUNGSVERFAHREN UND -VORRICHTUNG
PROCÉDÉ ET APPAREIL DE RESUSPENSION DE PLAQUETTES

(30) Priority: 03.05.2011 US 201161481959 P
(43) Date of publication of application: 12.03.2014
(73) Proprietor: Fenwal, Inc., Lake Zurich, IL 60047 (US)
(72) Inventor: BELT, Fletcher C., Bull Valley, IL 60098 (US); KAST, Michael J., Crystal Lake, IL 60012 (US); MIN, Kyungyoon, Kildeer, IL 60047 (US); RADWANSKI, Katherine, Desplaines, IL 60016 (US); CORK, William H., Lake Bluff, IL 60044 (US)
(74) Representative: Prins, Hendrik Willem
(86) International application number: PCT/US2012/036062
(87) International publication number: WO 2012/151243

(56) References cited:
- WO-A1-03/090795
- US-A- 5 427 695
- US-A- 5 427 695
- US-A- 5 690 815
- US-A1- 2002 143 244
- US-B1- 6 780 333

## Description

### Field of the Disclosure

The present application relates to the processing of blood components and, more particularly, to a method and apparatus for the resuspension of a concentrated blood component for storage and/or transfusion.

### Background

Continuous blood cell separation and collection is a well-known process for collecting desired blood components, such as red cells, platelets or plasma from a donor. Typically whole blood is withdrawn from a donor and directed into a centrifugal processing chamber to separate the whole blood into its various therapeutic components. This is usually carried out utilizing blood processing systems and methods comprising a durable centrifuge in association with a single-use, sterile fluid circuit that may include a processing chamber and associated storage containers, fluid flow tubing and the like. The processing chamber is usually mounted on a centrifuge rotor or bowl, which spins the chamber creating a centrifugal field, which separates the whole blood into its components based on their density.

A well-known and exemplary centrifugal blood processing system is the Amicus Separator, available from Fenwal Inc. of Lake Zurich, Illinois. The functional aspects of the Amicus Separator are disclosed in, e.g., U.S. Patent Nos. 6,312,607 and 6,582,349.

In the Amicus Separator, whole blood is typically separated into components in a two-stage process, using a belt-shaped processing container that has two separate processing chambers. The processing container is secured to a support in the form of a rotatable spool that is received within an outer bowl, such that the processing container is enclosed in an annular space between the spool and the bowl. The spool and bowl are rotated in unison about a common axis to centrifugally separate the whole blood into components. In the usual two-stage process for collecting platelets, whole blood is continuously separated into concentrated red cells and platelet rich plasma (PRP) in the first separation chamber, and the PRP is directed to the second centrifuge chamber for separation into platelet concentrate (PC) and platelet reduced (or poor) plasma (PPP). The red cells and PPP are returned to the donor or stored in collection containers, and the platelet concentrate accumulates in the second chamber.

After the desired components have been separated and the remaining components returned to the donor, the concentrated blood components (e.g. the PC) need to be prepared for storage and/or transfusion. This typically entails resuspension of the concentrated blood component in a liquid medium that promotes the preservation of the blood component during storage. In the case of concentrated platelets (PC), the resuspension medium may be plasma, a saline solution, or a combination thereof. Special saline solutions have been developed for resuspension and long-term storage of platelets that include components such as sodium citrate, acetate, phosphate, and potassium chloride. See e.g., U.S. Patent No. 6,063,624. Other solutions that have been developed for platelet storage that may also be useful in resuspension of platelets are available from Fenwal Inc. as Platelet Additive Solutions or PAS, PAS III or Intersol ®. Once the resuspension solution is added to the concentrated platelets, the collection container (which has been removed from the centrifuge and mounting spool) is often manually agitated to assist in resuspension. Sometimes the agitation must be vigorous to ensure that the concentrated platelets are completely resuspended and that there are no platelet aggregates or clumps of platelets that would make the platelets unsuitable for further processing or transfusion - because, for example, the aggregates may clog the macro-aggregate blood filters of the transfusion sets.

US patent no. US 5,427,695 discloses systems and methods for collecting and resuspending cellular-rich blood products like platelet concentrate. PCT patent application no. WO 03/090795 discloses an apparatus and method for irradiating and mixing fluid in containers. US patent no. US 5,690,815 describes an automated system for processing biological fluid and published US application US 2002/143244 details an in-vivo storage system.

### Summary of the Disclosure

The present invention provides the device for resuspending a concentrated blood component collected in a single-use processing chamber of Claim 1.

The invention also provides the method for collecting a concentrated blood component in a single-use processing chamber of claim 8 and the method for resuspending a concentrated blood component collected in a single-use processing chamber of claim 13. The dependent claims specify preferred but optional features.

The present subject matter has a number of aspects which may be used in various combinations and the disclosure of one or more specific embodiments is for the purposes of disclosure and description and not limitation. This summary only highlights a few of the aspects of this subject matter, and additional aspects are disclosed in the accompanying drawings and the following detailed description.

By way of the present application, a more efficient method is disclosed for more completely resuspending concentrated blood components, in particular concentrated platelets that are collected in a centrifugal processing system. Devices for mechanically agitating the collection container are also disclosed.

In one aspect of the disclosure, a device for resuspending the concentrated blood component collected in a single-use processing container or chamber is provided. The resuspension device comprises a support that is adapted to permit mounting of the single-use processing chamber thereto. A motor is operatively connected to the support for imparting a resuspending motion thereto, and a control is provided for actuating the motor. The controller may variously control one or more of the speed of motion imparted to the support, the time interval over which motion is imparted to the support, and the range of motion. The motion may be of any suitable resuspending motion, but preferably the motion is along an arcuate path and/or is reciprocatory.

In another aspect of the disclosure, the resuspension device is configured to impart a reciprocating arcuate motion to the support and its associated single-use processing chamber. A frequency of approximately 300 to 325 oscillations or reciprocatory movements over a period of time of approximately 1.5 to 2.5 minutes has been found effective for resuspending platelet concentrate collected in an Amicus processing chamber. Reciprocation is through an arc of less than 180°, and preferably less than about 90°, and more preferably between about 45° and 90°. The arc of reciprocation is preferably adjustable and, in one embodiment, the reciprocating motion is through an arc of about 57°.

In another aspect of the disclosure, a method for collecting a concentrated blood component in a single-use processing chamber is provided. The method includes the steps of mounting the single-use processing chamber to a rotatable support of a centrifugal collection system; introducing a suspension of blood components into the processing chamber and rotating the support to separate a desired blood component from the suspension until a sufficient amount of the desired blood component has been separated; introducing a resuspension solution to the single-use processing chamber containing the separated blood component; removing the rotatable support having the single-use processing chamber mounted thereto from the centrifugal collection system; mounting the support and its associated single-use processing chamber to a resuspension device; and activating the resuspension device to impart a resuspending motion to the support and its associated processing chamber for a period of time sufficient to resuspend the concentrated blood component in the resuspension solution.

In one aspect, a method is disclosed for resuspending a concentrated blood component collected in a single-use centrifugal processing that is secured to a rotatable support forming part of a centrifugal collection system. In connection with this aspect, the processing chamber is not required to be removed from the support prior to resuspension. A resuspension solution is introduced to the single-use processing chamber containing the concentrated blood component while the chamber remains secured to the support. The rotatable support having the single-use processing chamber secured thereto is removed from the centrifugal collection system and placed in association with an automated mechanical resuspension device. The resuspension device is then activated to impart a resuspension motion to the support and processing chamber for a period of time sufficient to resuspend the concentrated blood component in the resuspension solution.

In a further aspect of the disclosure, the concentrated blood component comprises platelets and the resuspension solution comprises a platelet additive solution or PAS. Additionally, plasma may be added to the single-use processing chamber in combination with the platelet additive solution for resuspension of the concentrated platelets. In a specific example, the ratio of platelet additive solution to plasma is 65:35.

### Brief Description of the Drawings

Fig. 1 is a perspective view of a centrifugal blood collection system that may be used in connection with the resuspension method disclosed herein.
Fig 2 is a side elevation view, with portions broken away to show detail, of the separation chamber of the centrifuge system of Fig. 1, with the bowl and support elements in their upright position for receiving a single-use blood processing chamber.
Fig. 3 is a side sectional view of the separation chamber of Fig. 2.
Fig. 4 is a perspective view of the support element of Figs. 2 and 3, with an associated blood processing chamber secured to it for use.
Fig. 5 is a perspective view of a first embodiment of an automated resuspension device according to the present disclosure.
Fig. 6 is a perspective view of a second embodiment of an automated resuspension device according to the present disclosure.
Fig. 7 is a fragmentary side elevation view, in partial cross-section, of a third embodiment of an automated resuspension device according to the present disclosure.

### Detailed Description

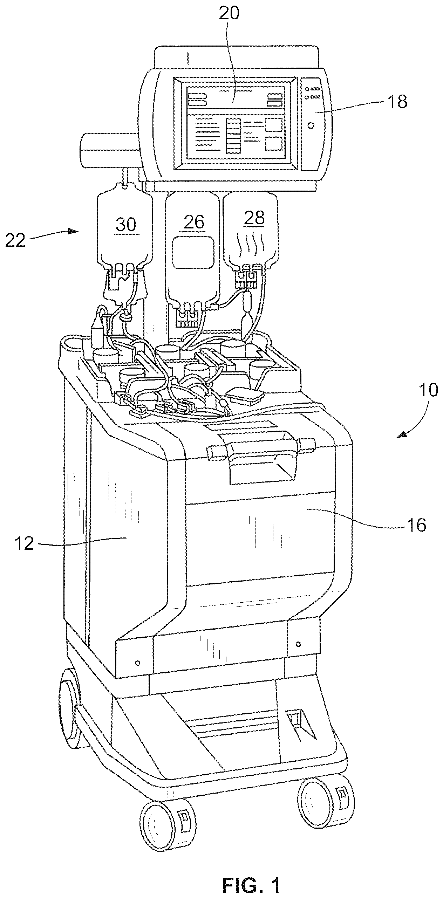

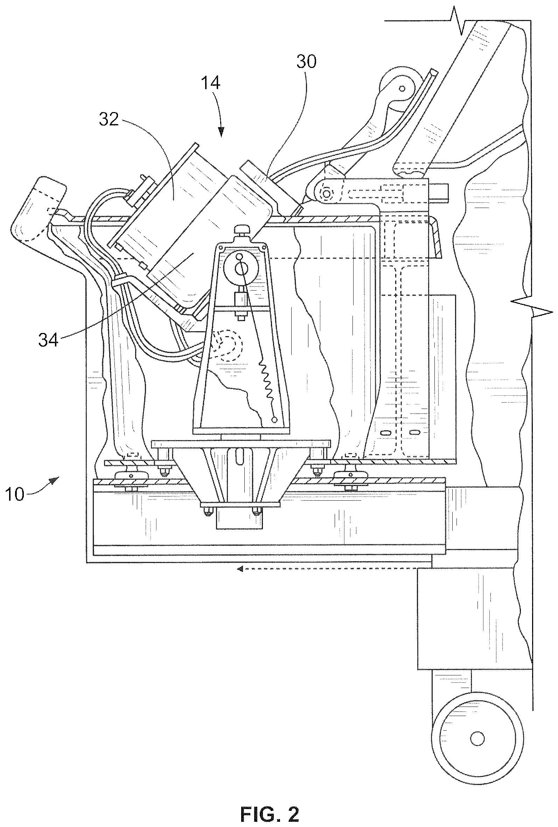

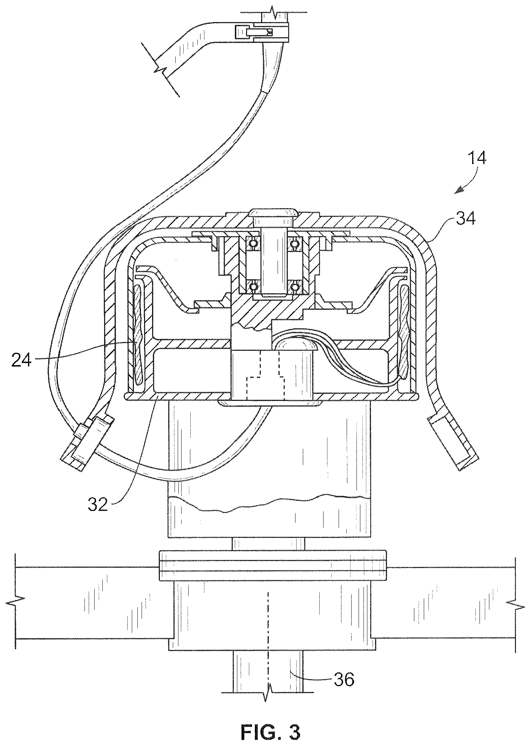

A more detailed description of the blood component resuspension methods performed and devices made in accordance with the present disclosure is set forth below. It should be understood that the description below of specific methods and devices is intended to be exemplary, and not exhaustive of all possible variations or applications. Thus, the scope of the disclosure is not intended to be limiting, and should be understood to encompass variations or embodiments that would occur to persons of ordinary skill. The invention is defined by the claims. Turning to the drawings, there is seen in Fig. 1 a perspective view of a centrifugal blood separation system, generally designated 10. As noted above, the Amicus Separator is exemplary of such a system 10. The system 10 includes a housing 12 for the centrifuge 14 (best seen in Figs. 2 and 3), including a door 16 through which access to the centrifuge 14 is obtained. A micro-processor based controller or control system 18 is supported above the housing 12 that includes a user interface in the form of touch screen 20, through which data can be input and operation of the centrifuge system 10 is controlled.

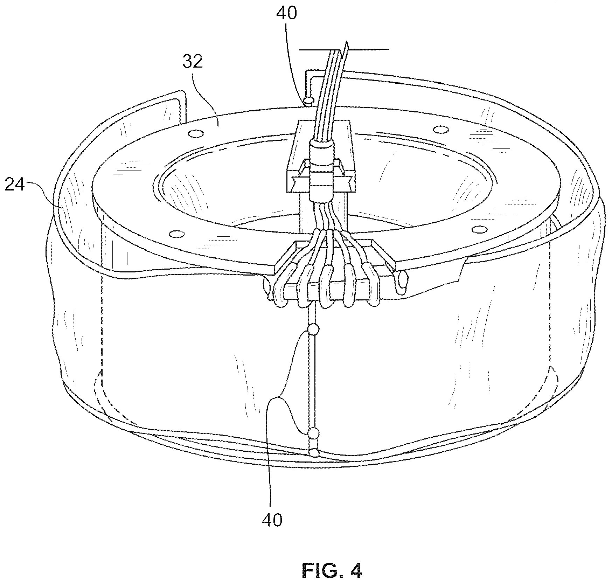

A single use/disposable collection kit, generally designated 22, is used in combination with the system 10. The collection kit 22 is typically made of a flexible plastic material and includes among other components, a processing container or chamber 24 (best seen in Fig. 4) that is mounted to the centrifuge 14. A tubing (not shown) and associated access needle are connected to the donor for withdrawing whole blood from the donor and introducing it into the processing chamber/container 24. Prefilled solution bags 26 and 28 for saline and anticoagulant are suspended above the centrifuge housing 12, as is a collection bag 30 for receiving, e.g., plasma that has been separated in the centrifuge from the whole blood.

Turning to Figs. 2 and 3, the centrifuge 14 is shown in greater detail. In pertinent part, the centrifuge 14 includes a support element in the form of spool 32 to which the processing chamber 24 is mounted. The spool 32 is removably inserted into a bowl element 34 with the processing chamber 24 wrapped about the spool element 32. In operation, the spool 32 and bowl 34 are rotated in unison about a common axis 36 (Fig. 3.)

The spool 32 and bowl 34 are mounted on a yoke 38 so as to be pivotable between an upright position (Fig. 2), in which the spool 32 extends at least partially out of the bowl 34 for attachment and/or removal of the processing chamber, and an inverted position (Fig. 3), in which the processing chamber 24 is enclosed between the spool 32 and the bowl 34 for rotation about the axis 36. In the illustrated embodiment, the processing chamber 24 is secured to the spool 32 by means of pins 40 (Fig. 4) on the spool 32 that engage cutouts on the processing chamber 24, although any suitable means for securing the chamber to a centrifuge support may be used.

Using a centrifugal separation system 10, such as that described above for processing whole blood to obtain a concentrated blood component in the processing chamber 24 is described in detail in the above-referenced U.S. patents, and will not be repeated here. Once the concentrated blood component has been obtained, the donor is disconnected from the collection kit, and the concentrated component is ready for further processing to facilitate its storage and future use.

It is often desirable to resuspend the concentrated blood component after collection. In accordance with the present disclosure, resuspension is achieved by introducing a resuspension solution into the processing chamber containing the concentrated blood component, e.g., platelet concentrate. Then, the rotatable support that has the processing chamber mounted thereto is removed from the centrifuge and mounted to a resuspension device. Leaving the processing chamber secured to the centrifuge support simplifies the resuspension task. The resuspension device is then activated for a period of time sufficient to resuspend the concentrated blood component in the resuspension solution.

It has been found that by adding a platelet additive solution to the concentrated platelets, platelet resuspension is accomplished more easily than resuspension in 100% plasma. The additive solution may be simply saline or a commercially available platelet additive solution and the percentage of additive solution in the total resuspension fluid in the chamber may range from 60% to 100%, with the remainder mostly plasma and platelets. Exemplary solutions useful for resuspending (and subsequent storage of platelets) include solutions such as those disclosed in U.S. Patent Nos.: 6,251,580; 6,566,046; 6,866,992 U.S. Patent Application Publication No. US2009/0191537 and PCT/US2012/032551. A combination of platelet additive solution and plasma in a ratio of about 2:1 or, more specifically, about 65:35 has been found effective, although other combinations of platelet additive solution and plasma (e.g., where the amount of plasma is reduced) may also be effective. Further, the additive solution used to resuspend the platelets may or may not be the same as the additive solution to store the platelets.

In keeping with another aspect of the disclosure, a resuspension device comprising an automated mechanical resuspender is provided onto which the support and its associated processing chamber can be mounted to automatically agitate the concentrated blood component and resuspension fluid to resuspend the blood component. It is believed that a reciprocatory, back and forth motion most efficiently resuspends the blood components without undue harm to the blood components.

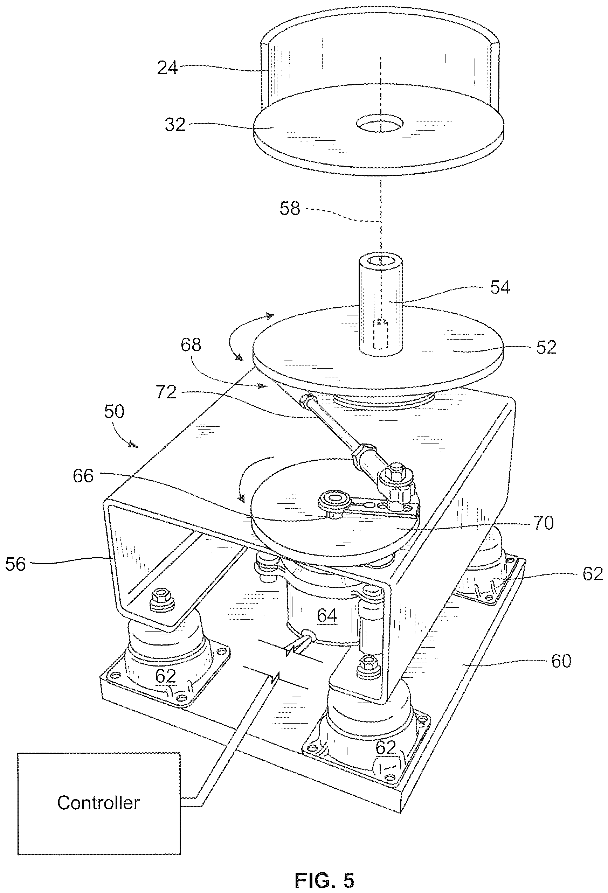

With reference to Fig. 5, there is seen a resuspension device generally designated 50 appropriate for use in the resuspension method described above. The resuspension device 50 comprises a mounting base, such as plate 52, with a spindle 54 extending therefrom, onto which the centrifuge or dedicated support or spool 32 can be securely mounted. Preferably the plate 52 and spindle 54 utilize the same attachment mechanisms that are used for securing the spool 32 thereto as does the centrifugal separator system 10, so that the procedure for attaching and removing the spool 32 to the resuspension device 50 should be readily familiar to the operator.

The plate 52 is mounted for rotation relative to a stationary support or frame 56 about an axis 58. It may be necessary to provide the plate 52 or spool 32 with a counterweight to sufficiently balance the combination of the plate 52 and spool 32 and reduce vibration resulting from the operation of the device 50. The frame 56 is mounted to a lower base 60 by means of commercially available vibration isolation mounts 62 in order to reduce vibration transmitted to the base.

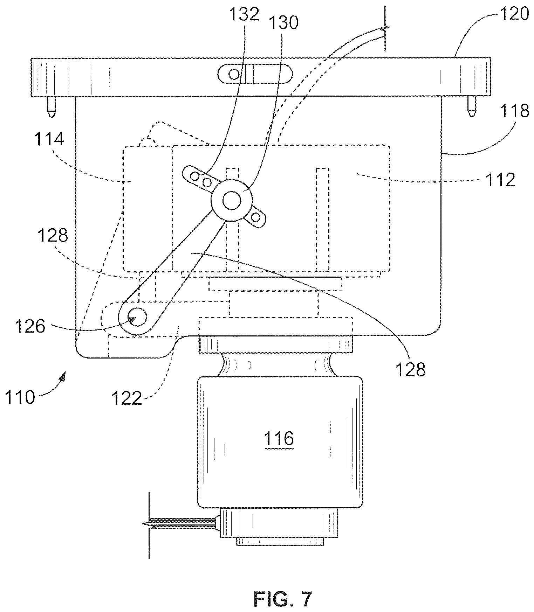

An electric motor 64 is secured to the frame 56 for rotating the plate 52/spool 32 about the axis 58. Various drive systems can be provided. As shown, the output shaft 66 of the motor 64 is connected to the plate 52/spindle 54 assembly by means of a crank arm assembly, generally designated 68, commonly referred to as a four-bar linkage. The four-bar linkage 68 converts the rotational movement of the output shaft 60 of the motor 64 to reciprocating arcuate or rotary movement of the plate 52/spindle 54 assembly about the axis 58 through a limited arc. The linkage 68 includes a crank arm or input link 70 in the form of a disk attached to the output shaft 66 of the motor 64, and a rocker arm or coupler link 72 pivotably connected to both the crank arm 70 and the plate 62, the latter forming the follower link of the four-bar linkage 68. Alternatively, the plate/spindle of the resuspension device may be mounted to a reversible, single axis, direct-acting motor, as shown in Fig. 7, which is described in greater detail below.

The frequency of reciprocation of the plate 52 can be varied by changing the rate of rotation of the output shaft 60 of the motor 64 through, e.g. a motor controller (not shown). The motor controller may also be used to control the period of time over which the plate/spool is rotated. A motor rotation speed of 300-325 rpm over a period of time of approximately 1.5 minutes to 2.5 minutes has been found effective for resuspending platelets in a platelet additive solution. Additionally, the arc through which the plate 52/spool 32 reciprocates can be varied by changing the length of either the input link 70 or the follower link (i.e., the radius of the plate 52). An arc of reciprocation of up to about 200° is contemplated, and is preferably adjustable within a range of from about 65° to about 160°. More preferably, the arc of reciprocation is less than 90°, such as between about 45° and 90°. In one example, reciprocation through an arc of about 57° has been found effective for resuspending concentrated platelets in a resuspension solution. The resuspension device is provided with a compression element, which is a roller that engages the processing chamber when the latter is secured to the outside of the spool. The roller acts to squeeze or compress the processing chamber to provide a further mixing action to its contents, thus enhancing the resuspension of the blood component contained therein.

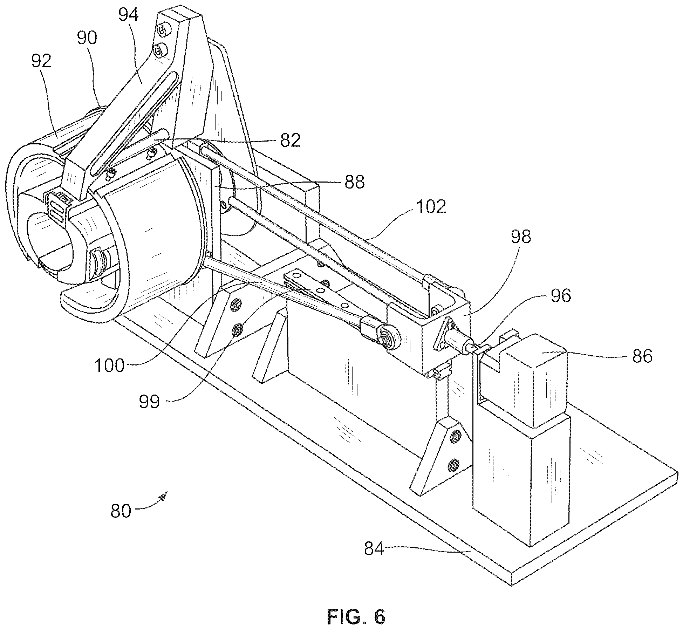

With reference to Fig. 6, a resuspension device 80 is seen having such a compression roller 82 associated therewith. The resuspension device 80 comprises a stationary support or frame 84 that supports both a drive motor 86, and a shaft 88 to which the spool plate 90 is mounted for rotation about an axis coincident with the axis of the shaft 88. The frame 84 may be supported on or within housing by means of vibration isolation mounts (not shown), similar to mounts 62 seen in Fig. 5. The compression roller 82 is mounted to the frame for engagement with the processing chamber when the latter is mounted to the spool 92, and the spool is attached to the shaft 88. As shown in Fig. 6, the compression roller 82 is rotatably mounted to an arm assembly 94, the arm assembly 94 being moveable so that the roller may be selectively brought into or out of engagement with the processing chamber. The moveable arm assembly 94 also preferably allows for varying the pressure with which the roller 82 engages the processing chamber.

It has been found that with the use of a compression roller the rate of reciprocation of the spool may be greatly reduced while still providing for resuspension of platelets within an acceptable period of time. In one embodiment, rotational speeds as low as 15 rpm have produced satisfactory results. These lower speeds help to reduce any vibration and noise generated by the use of resuspension device.

Once the roller 82 is brought into engagement with the processing chamber, the drive motor 86 may be activated to provide for relative reciprocatory motion between the spool 92 and the roller 82 to move the bloods components back and forth within the processing chamber for a time sufficient to affect resuspension.

As illustrated in Fig. 6, the compression roller 82/arm assembly 94 is also rotatably mounted to the shaft 88, such that the spool 92 and the compression roller 82/arm assembly 94 may be counter-rotated (i.e., simultaneously rotated in opposite directions) by the drive motor 86. To this end, the drive motor 86 comprises a linearly-moveable shaft 96 that includes a drive block 98 that, as illustrated, is moveable along a track 99. A first link 100 is pivotally connected to both the drive block 98 and the spool plate 90 for reciprocating the spool 92, while a second link 102 is pivotably connected to both the drive block 98 and the arm assembly 94 for reciprocating the compression roller 82. The links 100, 102 are oriented relative to the motor shaft 96 such that the spool plate 90 and the roller arm assembly 94 rotate in opposite directions about the axis of the shaft 88 upon reciprocatory, linear movement of the motor shaft 96. Alternatively, only one of the spool 92 and compression roller 82 needs to be reciprocated relative to the other. Consequently, one may be held stationary while the other is reciprocated. For example, the spool may be mounted to the drive shaft of a reversible, single axis, direct-acting motor, while the compression roller is stationary.

Turning to Fig. 7, there is seen a further embodiment of a resuspension device 110 according to the present disclosure, in which the mount 112 for the processing chamber reciprocates, while the compression roller 114 remains stationary. The resuspension device 110 comprises a single axis, direct-acting, reversible drive motor 116, with the mount/spool 112 being removably attached to the shaft of the motor. As illustrated, the mount/spool 112 is housed within a bowl 118 having a top mounting plate 120 for securing the bowl to a housing (not shown) for the resuspension device. The motor 116 is also mounted within the housing by means (not shown) that would occur to a person skilled in the art. For example, the motor may be suspended by the bowl 118 with the bowl 118 mounted to the housing by vibration-isolating mounts.

The motor 116 includes a mounting plate 122 disposed inside the bowl 118 to which the compression roller 114 is mounted for movement into and out of engagement with a processing chamber mounted to the spool 112. To this end, the compression roller 114 is rotatably mounted to a spindle or axle 124. The spindle/axle 124, in turn, is pivotably mounted to the plate 122 about an axis 126 so that it may be pivoted about the axis 126 to bring the compression roller into or out of its stationary operating position. A lever arm 128 is provided for pivoting the compression roller 114 about the axis 126. Preferably, the degree to which the compression roller can be pivoted into engagement with a processing chamber may be varied in order to control the amount of pressure the roller exerts on the processing chamber. To this end, the lever 128 preferably includes a handle 130 including an actuatable detent (not seen) that seats within, e.g. an aperatured track 132 that may be mounted to the interior or the bowl 118. The detent may be spring biased so that it extends through a selected aperture in the track 132 to lock the roller in position and may be retractable by means of a push button on the handle 130 to release the lever.

The degree of reciprocatory movement should be sufficient to allow the roller to move across the entire length of the processing chamber that is attached to the spool. An arc of reciprocation of approximately 200° has been found suitable in one embodiment. As can be appreciated, if the compression roller and spool are counter- rotated, an arc of reciprocation of each of approximately 100° in one direction is sufficient for providing a total arcuate movement of the roller across the processing chamber of approximately 200°.

In keeping with another aspect of the disclosure, the compression roller may be provided with a surface that enhances the mixing action as the roller moves across the surface of the processing chamber. To this end, the surface of the roller may have an irregular profile (as opposed to the roller comprising a right circular cylinder), and vary in its circumference along its length. For example, the roller may have longitudinal or radial ribs, protruding bumps or fingers, etc. Alternatively, or additionally, the surface of the roller may be deformable or of varying densities along its length or circumference. Preferably, the compression roller is easily replaceable, so that rollers of different profiles and materials may be substituted as required.

Thus, a highly efficient method and device for the resuspension of blood components has been disclosed. The description provided above is intended for illustrative purposes only, and is not intended to limit the scope of the invention to any particular embodiment described herein. Instead, the scope is to be as set forth in the appended claims.

## Claims

1. A device (50) for resuspending a concentrated blood component collected in a single-use processing chamber (24) comprising:
a support (32) adapted to permit mounting the single-use processing chamber (24) thereto;
a motor (64) operatively connected to the support (32) for imparting motion thereto;
a controller for actuating the motor (64); and
a compression element that is selectively engageable with the processing chamber (24) when mounted to the support (32), wherein the compression element is a roller (82).

2. The resuspension device (50) of claim 1 wherein the controller controls the speed of motion imparted to the support (32), the time interval over which motion is imparted to the support (32), and/or the range of motion imparted to the support (32).

3. The resuspension device (50) of claims 1 or 2 wherein the motor (64) imparts arcuate motion to the support (32).

4. The resuspension device (50) of any claims 1-3 wherein the motor (64) imparts reciprocatory motion to the support (32), and preferably the support (32) is reciprocated through an arc of less than 200°.

5. The resuspension device (50) of any of claims 1-4 wherein the motor (64) comprises a drive shaft (66) rotatable about a first axis, the support is rotatably mounted about a second axis, and a linkage (68) connects the drive shaft (66) to the support (32) to impart motion to the support about the second axis in response to rotation of the drive shaft about the first axis.

6. The resuspension device (50) of any of claims 1-4 wherein the motor (64) comprises a rotatable drive shaft connected to the support such that the drive shaft and support rotate in unison about a common axis.

7. The resuspension device (50) of any of claims 1-6 wherein the roller (82) has an irregular profile and/or a deformable surface of varying densities and the processing chamber (24) is preferably a single-use processing chamber removably secured to the support (32).

8. A method for collecting a concentrated blood component in a single-use processing chamber (24) comprising:
mounting the single-use processing chamber (24) to a rotatable support (32) of a centrifugal collection system (10);
introducing a suspension of blood components into the processing chamber (24) and rotating the support (32) to separate a desired blood component from the suspension until a sufficient amount of the desired blood component has been separated;
introducing a resuspension solution to the single-use processing chamber (24) containing the separated blood component;
removing the rotatable support (32) having the single-use processing chamber (24) mounted thereto from the centrifugal collection system (10);
mounting the rotatable support (32) and its associated single-use processing container (24) to a resuspension device (50); and
activating the resuspension device (50) to impart a resuspending motion to the rotatable support (32) and associated processing chamber (24) for a period of time sufficient to resuspend the concentrated blood component in the resuspension solution.

9. The method of claim 8 wherein the resuspension device (50) imparts a reciprocating arcuate motion to the support (32) and its associated single-use processing chamber (24), which preferably reciprocate at a rate of approximately from 300 to 325 times per minute for approximately from 1.5 to 2.5 minutes, and/or the reciprocating arcuate motion is through an arc of less than 180°.

10. The method of claim 8 or 9 wherein the concentrated blood component comprises the platelets and the resuspension solution comprises a platelet additive solution.

11. The method of any of claims 8-10 wherein plasma is added to the single-use processing container (24) in combination with the platelet additive solution for resuspension of the concentrated platelets, and preferably the percentage of platelet additive solution in the resuspension solution ranges from 60% to 100%.

12. The method of any of claims 8-11 wherein a compression element (82)is brought into contact with the processing chamber (24) after the support and processing chamber (24) are mounted in the resuspension device (50), wherein the compression element is a roller (82).

13. A method for resuspending a concentrated blood component collected in a single-use processing chamber (24) mounted to a rotatable support (32) of a centrifugal collection system (10) comprising:
introducing a resuspension solution to the single-use processing chamber (24) containing the concentrated blood component;
removing the rotatable support having the single-use processing chamber (24) mounted thereto from the centrifugal collection system (10);
mounting the rotatable support (32) and its associated single-use processing chamber (24) to a mechanical shaker; and
activating the shaker for a period of time sufficient to resuspend the concentrated blood component in the resuspension solution.

14. The method of claim 13 wherein the mechanical shaker imparts a reciprocating arcuate motion to the support and its associated single-use processing chamber (24), and preferably the support and its associated single-use processing chamber reciprocate at a rate of from approximately 300 to 325 times per minute for from approximately 1.5 minutes to 2.5 minutes, and/or the reciprocating arcuate motion is through an arc of less than about 200°.

15. The method of any of claims 13-14 wherein a compression element (82) is brought into engagement with the processing chamber (24) after the support and processing chamber (24) are mounted in the mechanical shaker, wherein the compression element is a roller (82).

## Patentansprüche

1. Verfahren zum Bereitstellen von Identifizierungsinformationen an einem oder mehreren Aufbewahrungsbehältern (132, 134, 136) eines Verarbeitungskits (130) für ein Blutprodukt während der Verarbeitung einer Menge von Vollblut, das diesem zugeordnete Spenderidentifikationsdaten aufweist, mit einem Blutverarbeitungssystem mit einer Benutzerschnittstelle (140, 142), wobei das Verfahren die Schritte aufweist:
Auffordern des Benutzers zum Laden des einen oder der mehreren Aufbewahrungsbehälter (132, 134, 136) des Verarbeitungskits (130) in die Blutverarbeitungsvorrichtung;
entweder Abrufen oder Auffordern des Benutzers zum Abrufen von dem Verarbeitungskit (130) zugeordneten Daten;
entweder Abrufen oder Auffordern des Benutzers zum Abrufen der dem Vollblut zugeordneten Spenderidentifikationsdaten;
Zuordnen der Spenderidentifikationsdaten zu dem einen oder den mehreren Aufbewahrungsbehältern (132, 134, 136) des Verarbeitungskits (130), wodurch Aufbewahrungsbehälterdaten generiert werden;
Verarbeiten des Vollbluts in ein Blutprodukt, das in dem einen oder mehreren Aufbewahrungsbehältern (132, 134, 136) des Verarbeitungskits (130) aufgenommen wird;
entweder Abrufen oder Auffordern des Benutzers zum Abrufen der Aufbewahrungsbehälterdaten und Vergleichen dieser mit den Spenderidentifikationsdaten für das Vollblut zur Verifizierung der Aufbewahrungsbehälterdaten; und
nach der Verifizierung der korrekten Identifikationsinformationen der Aufbewahrungsbehälterdaten, Auffordern des Benutzers zum Entfernen des einen oder der mehreren Aufbewahrungsbehälter (132, 134, 136) des Verarbeitungskits (130), wobei das Verfahren durchgeführt wird unter Verwendung einer programmierbaren Steuereinheit, die dazu ausgebildet ist, die verschiedenen Schritte des Verfahrens automatisch durchzuführen und/oder einen Benutzer dazu aufzufordern.

2. Verfahren nach Anspruch 1, wobei die Identifizierungsinformationen an dem einen oder den mehreren Aufbewahrungsbehältern in Barcode-Form vorliegen und wobei der Benutzer dazu aufgefordert wird, die dem Verarbeitungskit (130) zugeordneten Daten, die Spenderidentifikationsdaten und die Aufbewahrungsbehälterdaten abzurufen.

3. Verfahren nach Anspruch 1, wobei die Identifizierungsinformationen an dem einen oder den mehreren Aufbewahrungsbehältern mit einem dem einen oder den mehreren Aufbewahrungsbehältern zugeordneten RFID-Tag oder -Tags verknüpft sind und wobei die dem Verarbeitungskit zugeordneten Daten, die Spenderidentifikationsdaten und die Aufbewahrungsbehälterdaten automatisch von der programmierbaren Steuereinheit abgerufen werden.

4. Verfahren nach Anspruch 1 bis 3, bei dem das Vollblut in einem Sammelbehälter (128) enthalten ist, der Spenderidentifikationsdaten aufweist, die damit in Form einer scanbaren Spender-ID des Sammelbehälters (128) und/oder in einem RFID-Tag am Sammelcontainer (128) verknüpft sind und Teil eines integrierten Systems (130) sind, das den einen oder die mehreren Aufbewahrungsbehälter (132, 134, 136) des Verarbeitungskits (130) enthält.

5. Verfahren nach Anspruch 1 bis 3, bei dem das Vollblut in einem Sammelbehälter (128) enthalten ist, der diesem in Form einer scanbaren Spender-ID des Sammelbehälters (128) und/oder in einem RFID-Tag am Sammelbehälter (128) zugeordnete Spenderidentifikationsdaten aufweist und anfangs von dem einen oder mehreren Aufbewahrungsbehältern (132, 134, 136) des Verarbeitungskits (130) getrennt ist und mit dem einen oder den mehreren Aufbewahrungsbehältern (132, 134, 136) des Verarbeitungskits (130) nach Abruf der dem Sammelbehälter zugeordneten Daten verbunden ist, wobei die Daten die Spenderidentifikationsdaten enthalten.

6. Verfahren nach Anspruch 2, wobei:
- das Auffordern des Benutzers zum Abrufen der Spenderidentifikationsdaten und der mit dem Verarbeitungskit verknüpften Daten das Auffordern des Benutzers zum Scannen einer Barcode-ID des Verarbeitungskits und der Spender-ID des Sammelbehälters ist, und
- die Zuordnung der Spenderidentifikationsdaten dadurch ausgeführt ist, dass das System zum Ausdrucken wiederholter Barcode-Label mit der Zuordnung der Spenderidentifikationsdaten zu dem einen oder den mehreren Aufbewahrungsbehältern (132, 134, 136) auf einem Drucker ausgebildet ist, und das System dem Benutzer zur Anbringung der gedruckten Barcode-Label an den Aufbewahrungsbehältern auffordert.

7. Verfahren nach Anspruch 3 bis 4, wobei die Zuordnung der Spenderidentifikationsdaten zu dem einen oder den mehreren Aufbewahrungsbehältern (132, 134, 136) dergestalt ausgeführt wird, dass das System Spenderidentifikationsdaten auf dem den Verarbeitungskits-Aufbewahrungsbehältern zugeordneten RFID-Tag oder -Tags automatisch repliziert.

8. Verfahren nach Anspruch 4 bis 5, wobei die Identifikationsdaten für die Sammelbehälter eines oder mehrere von einer Spender-ID, Behältertyp, Ablaufdatum, Spendezeit, gespendetes Volumen, Krankenschwester-ID und Spendeort aufweist.

9. Blutverarbeitungssystem (138) zur Verarbeitung von Vollblut oder einer Vollblutkomponente, die in einem Ausgangsbehälter (128) enthalten ist, wobei das Verarbeitungssystem dazu ausgestaltet ist, einen Verarbeitungsfluss-Kreislauf, der einen oder mehrere Aufbewahrungsbehälter (132, 134, 136) enthält, zu empfangen und eine Steuereinheit mit einer Benutzerschnittstelle (140, 142) enthält, die zur - Vereinfachung der Bereitstellung von Identifizierungsinformationen an dem einen oder den mehreren Aufbewahrungsbehältern (132, 134, 136) des Verarbeitungskreislaufes (130) basierend auf den dem Ausgangsbehälter (128) zugeordneten Spenderidentifikationsdaten programmiert ist, wobei die Steuereinheit dazu ausgestaltet ist:
den Benutzer zum Laden des einen oder der mehreren Aufbewahrungsbehälter (132, 134, 136) des Verarbeitungskreislaufes in das Blutverarbeitungssystem aufzufordern;
die dem Verarbeitungskreislauf zugeordneten Daten entweder abzurufen oder den Benutzer zum Abrufen der dem Verarbeitungskreislauf zugeordneten Daten aufzufordern;
die dem Ausgangsbehälter (128) zugeordneten Daten entweder abzurufen oder den Benutzer zum Abrufen der dem Verarbeitungskreislauf zugeordneten Daten aufzufordern;
Spenderinformationsdaten dem einen oder den mehreren Aufbewahrungsbehältern (132, 134, 136) zuzuordnen, wodurch Aufbewahrungsbehälterdaten generiert werden; das Fluid im Ausgangsbehälter (128) in ein Blutprodukt zu verarbeiten, das in dem einen oder den mehreren Aufbewahrungsbehältern (132, 134, 136) des Verarbeitungskreislaufes (130) aufgenommen wird;
die Aufbewahrungsbehälterdaten entweder abzurufen oder den Benutzer zum Abrufen der Aufbewahrungsbehälterdaten aufzufordern und diese mit den Spenderidentifikationsdaten zu vergleichen, um die Aufbewahrungsbehälterdaten zu verifizieren; und
nach der Verifizierung der korrekten Identifikationsinformationen der Aufbewahrungsbehälterdaten, den Benutzer zum Entfernen des einen oder der mehreren Aufbewahrungsbehälter (132, 134, 136) des Verarbeitungskits (130) aufzufordern.

10. Blutverarbeitungssystem nach Anspruch 9, wobei die Spenderidentifikationsdaten und die Identifizierungsinformationen an dem einen oder den mehreren Aufbewahrungsbehältern in Barcode-Form vorliegen, und wobei die Steuereinheit dazu ausgestaltet ist, den Benutzer zum Abrufen der mit dem Verarbeitungskreislauf verknüpften Daten, der Spenderidentifikationsdaten und der Aufbewahrungsbehälterdaten aufzufordern.

11. Blutverarbeitungssystem nach Anspruch 10, wobei die Steuereinheit weiterhin dazu ausgestaltet ist, wenigstens ein Label zur Anbringung an dem einen oder den mehreren Aufbewahrungsbehältern zu generieren.

12. Blutverarbeitungssystem nach Anspruch 9, wobei die Identifizierungsinformationen an dem einen oder den mehreren Aufbewahrungsbehältern einem dem einen oder den mehreren Aufbewahrungsbehältern zugeordneten RFID-Tag oder -Tags zugeordnet sind und die Spenderidentifikationsdaten einer RFID am Ausgangsbehälter (128) zugeordneten werden, die Steuereinheit dazu ausgestaltet sind, und wobei die dem Verarbeitungskreislauf zugeordneten Daten, die Spenderidentifikationsdaten und die Speicherbehälterdaten abzurufen.

13. Blutverarbeitungssystem nach Anspruch 12, wobei das System zur automatischen Replikation der Spenderidentifikationsdaten auf dem dem Ausgangsbehälter (128) zugeordneten RFID-Tag auf die den Aufbewahrungsbehältern (132, 134, 136) zugeordneten RFID-Tag oder -Tags ausgestaltet ist, wodurch eine Zuordnung der Spenderidentifikationsdaten zu dem einen oder den mehreren Aufbewahrungsbehältern (132, 134, 136) ermöglicht wird.

14. Blutverarbeitungssystem nach einem der Ansprüche 9 bis 13, wobei die Steuereinheit in einer Blutverarbeitungseinrichtung oder in einer sterilen Verbindungsvorrichtung zum Verbinden eines Ausgangsbehälters mit einem Verarbeitungsfluidfließ-Kreislauf enthalten ist.

15. Blutverarbeitungssystem nach Anspruch 9 bis 14, wobei die Spenderidentifikationsdaten, die Identifizierungsinformationen an den Aufbewahrungsbehältern ebenso als Barcode-Label ausgebildet sind und das System zur Nutzung von sowohl Barcode als auch als RFID als redundante Systeme ausgebildet ist.

## Revendications

1. Procédé de fourniture d'informations d'identification sur un ou plusieurs conteneurs de stockage (132, 134, 136) d'un kit de traitement (130) pour un produit sanguin au cours du traitement d'une quantité de sang total associée à des données d'identification de donneur, avec un dispositif de traitement de sang comportant une interface d'utilisateur (140, 142), dans lequel le procédé comprend les étapes de :
demande à l'utilisateur de charger le ou les conteneurs de stockage (132, 134, 136) du kit de traitement (130) sur le dispositif de traitement du sang ;
soit extraction soit demande à l'utilisateur d'extraire des données associées au kit de traitement (130) ;
soit extraction, soit demande à l'utilisateur d'extraire les données d'identification de donneur associées au sang total ;
association des données d'identification de donneur au ou aux conteneurs de stockage (132, 134, 136) du kit de traitement(130), produisant avec celles-ci des données de conteneur de stockage ;
traitement du sang total en un produit sanguin reçu dans le ou les conteneurs de stockage (132, 134, 136) du kit de traitement (130) ;
soit extraction, soit demande à l'utilisateur d'extraire les données de conteneur de stockage et comparaison de ces dernières avec les données d'identification de donneur pour le sang total afin de vérifier les données de conteneur de stockage ; et
après vérification des informations d'identification correctes des données de conteneur de stockage, demande à l'utilisateur de séparer le ou les conteneurs de stockage (132, 134, 136) du kit de traitement(130),
dans lequel le procédé est mis en oeuvre en utilisant une unité de commande programmable configurée de manière à exécuter automatiquement et/ou en demandant à un utilisateur d'exécuter les différentes étapes du procédé.

2. Procédé selon la revendication 1, dans lequel lesdites informations d'identification sur le ou les conteneurs de stockage sont sous la forme d'un code à barres, et dans lequel il est demandé à l'utilisateur d'extraire les données associées au kit de traitement (130), les données d'identification de donneur et les données de conteneur de stockage.

3. Procédé selon la revendication 1, dans lequel lesdites informations d'identification sur le ou les conteneurs de stockage sont associés à une ou des étiquettes RFID associées au ou aux conteneurs de stockage, et dans lequel les données associées au kit de traitement, les données d'identification de donneur et les données de conteneur de stockage sont extraites automatiquement par l'unité de commande programmable.

4. Procédé selon les revendications 1 à 3, dans lequel le sang total est contenu dans un conteneur de collecte (128) qui est associé à des données d'identification de donneur sous la forme d'un identifiant (ID) de donneur pouvant être lu du conteneur de collecte (128) et/ou sur une étiquette RFID sur le conteneur de collecte (128) et est une partie d'un dispositif intégré (130) comportant le ou les conteneurs de stockage (132, 134, 136) du kit de traitement (130).

5. Procédé selon les revendications 1 à 3, dans lequel le sang total est contenu dans un conteneur de collecte (128) qui comporte des données d'identification de donneur qui lui sont associées sous la forme d'un identifiant ID de donneur du conteneur de collecte (128) pouvant être scanné et/ou sur une étiquette RFID sur le conteneur de collecte (128) et est initialement séparé du ou des conteneurs de stockage (132, 134, 136) du kit de traitement (130) et est relié au ou aux conteneurs de stockage (132, 134, 136) du kit de traitement (130) après extraction des données associées au conteneur de collecte, lesdites données comportant les données d'identification de donneur.

6. Procédé selon la revendication 2, dans lequel
la demande à l'utilisateur d'extraire les données d'identification de donneur et les données associées au kit de traitement est la demande à l'utilisateur de lire un identifiant à code à barres du kit de traitement et l'identifiant de donneur d'un conteneur de collecte, et
l'association des données d'identification de donneur est mise en oeuvre en ce que le dispositif est configuré afin d'imprimer, sur une imprimante, des étiquettes à code à barres dupliquées de l'association des données d'identification de donneur avec le ou les conteneurs de stockage (132, 134, 136), et en ce que le dispositif demande à l'utilisateur d'appliquer les étiquettes à code à barres imprimées sur les conteneurs de stockage.

7. Procédé selon les revendications 3 et 4, dans lequel l'association des données d'identification de donneur avec le ou les conteneurs de stockage (132, 134, 136) est mise en oeuvre en ce que le dispositif duplique automatiquement les données d'identification de donneur sur la ou les étiquettes RFID associées aux conteneurs de stockage de kit de traitement.

8. Procédé selon les revendications 4 et 5, dans lequel les données d'identification pour le conteneur de collecte comprennent un ou plusieurs parmi un identifiant de donneur, un type de conteneur, une date d'expiration, une heure de collecte, un volume de collecte, un identifiant de soignant et un site de collecte.

9. Dispositif de traitement de sang (138) destiné à traiter du sang total ou un composant de sang total contenu dans un conteneur source (128), le dispositif de traitement étant configuré de manière à recevoir un circuit d'écoulement de traitement comportant un ou plusieurs conteneurs de stockage (132, 134, 136) et comportant une unité de commande avec une interface d'utilisateur (140, 142) programmée afin de faciliter la fourniture d'informations d'identification sur le ou les conteneurs de stockage (132, 134, 136) du circuit de traitement (130) sur la base de données d'identification de donneur associées au conteneur source (128), l'unité de commande étant configurée de manière à :
demander à l'utilisateur de charger le ou les conteneurs de stockage (132, 134, 136) du circuit de traitement sur le dispositif de traitement de sang ;
soit extraire, soit demander à l'utilisateur d'extraire les données associées au circuit de traitement (130) ;
soit extraire, soit demander à l'utilisateur d'extraire les données d'identification de donneur associées au conteneur source (128) ;
associer les données d'identification de donneur au ou aux conteneurs de stockage (132, 134, 136), produisant avec celles-ci des données de conteneur de stockage ;
traiter le fluide dans le conteneur source (128) en un produit sanguin qui est reçu dans le ou les conteneurs de stockage (132, 134, 136) du circuit de traitement (130) ;
soit extraire, soit demander à l'utilisateur d'extraire les données de conteneur de stockage et les comparer aux données d'identification de donneur afin de vérifier lesdites données de conteneur de stockage, et
après vérification des informations d'identification correctes des données de conteneur de stockage, demander à l'utilisateur de séparer le ou les conteneurs de stockage (132, 134, 136) du kit de traitement (130).

10. Dispositif de traitement de sang selon la revendication 9, lesquelles données d'identification de donneur et lesquelles informations d'identification sur le ou les conteneurs de stockage sont sous la forme d'un code à barres, et dans lequel l'unité de commande est configurée de manière à demander à l'utilisateur d'extraire les données associées au circuit de traitement, les données d'identification de donneur et les données de conteneur de stockage.

11. Dispositif de traitement de sang selon la revendication 10, dans lequel l'unité de commande est en outre configurée de manière à produire au moins une étiquette afin de fixer celle-ci sur un ou plusieurs conteneurs de stockage.

12. Dispositif de traitement de sang selon la revendication 9, dans lequel lesdites informations d'identification sur le ou les conteneurs de stockage étant associées à une ou des étiquette RFID associées au ou aux conteneurs de stockage et les données d'identification de donneur étant associées à une étiquette RFID sur le conteneur source (128), l'unité de commande étant configurée de manière à extraire les données associées aux circuit de traitement, les données d'identification de donneur et les données de conteneur de stockage.

13. Dispositif de traitement de sang selon la revendication 12, dans lequel le dispositif est configuré de manière à dupliquer automatiquement les données d'identification de donneur sur l'étiquette RFID associée au conteneur source (128) sur l'étiquette ou les étiquettes RFID associées aux conteneurs de stockage (132, 134, 136), permettant ainsi l'association des données d'identification de donneur au ou aux conteneurs de stockage (132, 134, 136).

14. Dispositif de traitement de sang selon l'une quelconque des revendications 9 à 13, dans lequel l'unité de commande est contenue à l'intérieur d'un dispositif de traitement de sang, ou à l'intérieur d'un dispositif de liaison stérile afin d'assurer la liaison d'un conteneur source à un circuit d'écoulement de fluide de traitement.

15. Dispositif de traitement de sang selon l'une quelconque des revendications 9 à 14, dans lequel les données d'identification de donneur, les informations d'identification sur les conteneurs de stockage sont aussi mises en oeuvre sous forme d'étiquettes à code à barres et le dispositif est configuré de manière à utiliser à la fois le code à barres et l'identifiant RFID en tant que dispositifs redondants.
